# EUROPÄISCHE PATENTSCHRIFT

(11) **EP 0 369 400 B1**
(45) Veröffentlichungstag und Bekanntmachung des Hinweises auf die Patenterteilung: **13.01.1993**
(21) Anmeldenummer: 89121066.8
(22) Anmeldetag: 14.11.1989
(51) Int. Cl.: A61D 7/00, A61M 31/00

(54) **Verfahren zum Herstellen eines intravaginalen Applikationssystems**
Method of evolving an intravaginal application system
Méthode de fabrication pour système d'application intra-vaginale

(30) Priorität: 17.11.1988 DE 3838815
(43) Veröffentlichungstag der Anmeldung: 23.05.1990
(73) Patentinhaber: HOECHST AKTIENGESELLSCHAFT, 65926 Frankfurt am Main (DE)
(72) Erfinder: Hiller, Dietrich, Dr., D-6200 Wiesbaden (DE); Hornykiewytsch, Theophil, Dr., D-6230 Frankfurt am Main 80 (DE)

(56) Entgegenhaltungen:
- EP-A- 0 013 949
- EP-A- 0 086 530
- FR-A- 2 162 628
- FR-A- 2 285 895
- FR-A- 2 603 191
- US-A- 4 043 339

## Beschreibung

Gegenstand der Erfindung ist ein Verfahren zum Herstellen eines intravaginalen Applikationssystems zum kontrollierten Freisetzen von Substanzen, wobei das Applikationssystem aus einem elastischen Kern mit einem die Substanzen enthaltenden Umhüllung besteht. Die Erfindung betrifft ferner das Applikationssystem selbst.

Therapeutische Systeme für die Vagina eines Tieres bestehen im allgemeinen aus einem wirkstoffreien Kern, der vor allem für die Stabilität und Flexibilität des Systems verantwortlich ist, und einer polymeren Umhüllung - der Matrix - die die abzugebenden Wirkstoffe enthält und deren Freisetzungsrate bestimmt. Nach der DE 37 28 671 A1 ist ein Verfahren zum Herstellen einer Vorrichtung der eingangs genannten Art bekannt, bei dem der vorgeformte Kern aus Polyamid in einer Spritzgußform entsprechend zentriert mit spritzgießbarem Siliconkautschuk, der Progesteron enthält, ummantelt wird. Der Kautschuk wird bei Temperaturen von 180°C bis 230°C gehärtet. Bei dieser Temperatur schmilzt das eingemischte Progesteron, dessen Schmelztemperatur für die α-Modifikation ca. 130°C und für die β-Modifikation ca. 121°C beträgt. Nachteilig ist, daß beim Abkühlen der Progesteronschmelze auf Raumtemperatur bevorzugt die β-Modifikation entsteht. Diese tritt teilweise unkontrolliert aus der Matrix aus und setzt sich als puderartige Kristallschicht auf der Oberfläche des Siliconkautschuks ab. Daneben kristallisiert sie teilweise in die α-Modifikation um. Durch beide Ereignisse ändert sich die Freisetzungsrate der Vorrichtung während der Lagerung ständig. Die hohe Verarbeitungstemperatur schränkt außerdem die Auswahl eines für die Herstellung des wirkstoffreien Kernes geeigneten Materials stark ein. Das Material muß relativ hohe Warmfestigkeit aufweisen, damit beim Härten des flüssigen Siliconkautschuks keine Verformungen des Kerns auftreten.

Hier will die Erfindung Abhilfe schaffen.

Die Erfindung löst die Aufgabe durch ein Verfahren zum Herstellen eines intravaginalen Applikationssystems, das dadurch gekennzeichnet ist, daß man in ein Formteil einer geteilten Form ein die Substanzen enthaltendes Fell aus Siliconkautschuk einlegt, den Kern des Systems auf dem Fell anordnet und mit einem zweiten Fell bedeckt, danach die Form schließt und bei einer Temperatur von höchstens 120°C vulkanisiert.

Als Fell kann ein Gemisch aus Siliconkautschuk und Substanzen verwendet werden, die aus therapeutisch wirksamen Komponenten und die Eigenschaften des Silikons beeinflussende Komponenten bestehen. Als therapeutisch wirksame Komponente kann α-Progesteron verwendet werden. Als Komponenten, die die Eigenschaften des Siliconkautschuks beeinflussen können, können Siliconöl, Laurinsäureester, Glycerin, Siliciumdioxid, Capryl-Caprinsäure-Triglyceride und/oder deren Ester verwendet werden. Um einen guten Sitz, geringe Verlustrate und möglichst geringe Schleimhautreizung in der Vagina zu gewährleisten, sollte für das System ein Kern mit einer Rückstellkraft von 5 bis 15 N verwendet werden, der sich voll an die Schleimhaut anschmiegt. Kernmaterial mit der geforderten Rückstellkraft hat Dicken von 2 bis 15 mm.

Das System selbst ist dadurch gekennzeichnet, daß auf einem Kern mit einer Rückstellkraft von 5 bis 15 N ein Fell aus Siliconkautschuk mit einer Sprungtemperatur zwischen 60 und 120°C angeordnet ist, das α-Progesteron und den Siliconkautschuk beeinflussende Substanz enthält.

Die durch die Erfindung erreichten Vorteile sind im wesentlichen darin zu sehen, daß beim Härten des Siliconkautschuks der Wirkstoff nicht schmilzt, so daß sich keine unterschiedlichen Modifikationen mit unterschiedlichen Freisetzungsraten bilden können. Außerdem kann als Kern statt des relativ unelastischen Polyamids, das zwecks Erhöhung seiner Elastizität mit elastischen Gelenken versehen werden muß, das elastischere, weniger warmfeste Polypropylen verwendet werden, das im Gegensatz zu Polyamid nicht entfeuchtet werden muß.

Für das erfindungsgemäße Applikationssystem eignen sich insbesondere Kerne aus Polypropylen mit T-förmiger Gestalt. Je nach Verwendungszweck ist der Schaft (Steg) 50 bis 140 mm hoch und der Flansch 40 bis 200 mm breit. Die Länge von Steg und Flansch kann 5 bis 30 mm und die Dicke 2 bis 15 mm betragen.

Bei dem erfindungsgemäßen Verfahren wird ein fester Siliconkautschuk verwendet, in den auf einem Walzwerk die Substanzen, wie vor allem Wirkstoffe, z.B. Progesteron, Stoffe, die die Freigabe der Wirkstoffe aus der Siliconmatrix beeinflussen (europäisches Patent 0 013 949), und Stoffe, die die physikalisch-mechanischen Eigenschaften der Siliconmatrix beeinflussen eingewalzt. Dieser Vorgang dauert ca. 15 bis 30 Minuten. Ist eine der einzuwalzenden Komponenten flüssig, kann diese vor dem Einwalzen mit Siliciumdioxid oder einem anderen inerten Mittel, z.B. einem Pigment, zu einer Paste angeteigt werden. Je nach Spaltbreite des Walzwerkes entsteht ein unterschiedlich dickes Fell. In ein Formteil einer beheizbaren geteilten Form wird ein entsprechendes Fell von 2 bis 10 mm Dicke eingelegt, der Kern darauf angeordnet und mit einem zweiten Fell gleicher Dicke abgedeckt. Nach Schließen der Form wird diese auf Vulkanisationstemperatur erwärmt, diese Temperatur sollte bei Verwendung von Progesteron zwischen 70°C und 120°C, vorzugsweise zwischen 80°C und 110°C liegen. Es muß also ein Siliconkautschuk ausgewählt werden, dessen Vulkanisationstemperatur (Sprungtemperatur) in diesem Bereich liegt. Die Vulkanisationszeit beträgt ca. 4 bis 8 Minuten.

## Patentansprüche

1. Verfahren zum Herstellen eines intravaginalen Applikationssystems zum kontrollierten Freisetzen von Substanzen, wobei das Applikationssystem aus einem elastischen Kern mit einer die Substanzen enthaltenden Umhüllung besteht, dadurch gekennzeichnet, daß man in ein Formteil einer geteilten Form ein die Substanzen enthaltendes Fell aus Siliconkautschuk einlegt, den Kern des Systems auf dem Fell anordnet und mit einem zweiten Fell bedeckt, danach die Form schließt und bei einer Temperatur von höchstens 120°C vulkanisiert.

2. Verfahren nach Anspruch 1, dadurch gekennzeichnet, daß als Fell ein Gemisch aus Siliconkautschuk und Substanzen verwendet wird, die aus therapeutisch wirksamen Komponenten und die Eigenschaften des Siliconkautschuks beeinflussenden Komponenten bestehen.

3. Verfahren nach Anspruch 2, dadurch gekennzeichnet, daß als therapeutisch wirksame Komponente α-Progesteron verwendet wird.

4. Verfahren nach Anspruch 2, dadurch gekennzeichnet, daß als die Eigenschaften des Siliconkautschuks beeinflussende Komponenten Siliconöl, Laurinsäureester, Glycerin, Siliciumdioxid, Capryl-Caprinsäure-Triglyceride und/oder deren Ester verwendet werden.

5. Verfahren nach Anspruch 1, dadurch gekennzeichnet, daß ein Kern mit einer Rückstellkraft von 5 bis 15 N auf dem Fell angeordnet wird.

6. Applikationssystem zum kontrollierten Freisetzen von Substanzen bestehend aus einem elastischen Kern mit einer die Substanzen enthaltenden Umhüllung, dadurch gekennzeichnet, daß auf einem Kern mit einer Rückstellkraft von 5 bis 15 N ein die Substanzen enthaltendes Fell aus Siliconkautschuk mit einer Sprungtemperatur zwischen 60 und 120°C angeordnet ist, das α-Progesteron und die Eigenschaften des Siliconkautschuks modifizierende Substanzen enthält.

## Revendications

1. Procédé pour fabriquer un système, destiné à l'application intravaginale, qui permet d'ajuster la libération de substances, le système d'application étant constitué d'un noyau élastique muni d'un enrobage renfermant les substances, caractérisé en ce qu'on introduit une pellicule en caoutchouc silicone, contenant les substances, dans une moitié de moule, on place le noyau du système sur la pellicule et on le recouvre d'une deuxième pellicule, après quoi on ferme le moule et on effectue le durcissement à une température valant, au plus, 120°C.

2. Procédé selon la revendication 1, caractérisé en ce que, comme pellicule, on utilise un mélange de caoutchouc silicone et de substances constituées de composants actifs du point de vue thérapeutique et de composants exerçant une influence sur les propriétés du caoutchouc silicone.

3. Procédé selon la revendication 2, caractérisé en ce qu'on utilise l'α-progestérone comme composant actif du point de vue thérapeutique.

4. Procédé selon la revendication 2, caractérisé en ce que, comme composants exerçant une influence sur les propriétés du caoutchouc,silicone, on utilise de l'huile de silicone, un laurate, de la glycérine, du dioxyde de silicium, des triglycérides caprylo-capriques et/ou des esters de ceux-ci.

5. Procédé selon la revendication 1, caractérisé en ce qu'on place un noyau ayant une force de rappel valant de 5 à 15 N sur la pellicule.

6. Système d'application permettant d'ajuster la libération de substances, constitué d'un noyau élastique muni d'un enrobage renfermant les substances, caractérisé en ce que, sur un noyau ayant une force de rappel valant de 5 à 15 N, on place une pellicule en caoutchouc silicone renfermant les substances, ayant une température de discontinuité comprise entre 60 et 120°C, qui contient de l'α-progestérone et des substances modifiant les propriétés du caoutchouc silicone.

## Claims

1. A process for preparing an intravaginal application system for the controlled release of substances, the application system comprising an elastic core with a sheathing containing the substances, which comprises inserting a rilled sheet of silicone rubber containing the substances in one mold component of a split mold, arranging the core of the system on the rilled sheet and covering it with a second milled sheet, then closing the mold and vulcanizing at a maximum temperature of 120°.

2. The process as claimed in claim 1, wherein a mixture of silicone rubber and substances comprising therapeutically effective components and components which affect the properties of the silicone rubber is used as rilled sheet.

3. The process as claimed in claim 2, wherein α-progesterone is used as therapeutically effective component.

4. The process as claimed in claim 2, wherein silicone oil, esters of lauric acid, glycerol, silicon dioxide, caprylic/capric acid triglycerides and/or esters of these acids are used as components which affect the properties of the silicone rubber.

5. The process as claimed in claim 1, wherein a core with a resilience of 5 to 15 N is arranged on the milled sheet.

6. An application system for the controlled release of substances, comprising an elastic core with a sheathing containing the substances, wherein a milled sheet of silicone rubber with a kick-off temperature of between 60 and 120°C, and containing the substances is arranged on a core having a resilience of 5 to 15 N, the milled sheet containing α-progesterone and substances which modify the properties of the silicone rubber.
